# EUROPEAN PATENT APPLICATION

(11) **EP 0 653 635 A1**
(43) Date of publication of application: **17.05.1995**
(21) Application number: 94116822.1
(22) Date of filing: 25.10.1994
(51) Int. Cl.: G01N 33/52, G01N 33/53, G01N 33/543, A61K 9/24, A01N 25/34

(54) **Physiologically active substance fixed sheet, process for its production, method for its storage, and method of supplying physiologically active substance through the sheet**

(30) Priority: 17.11.1993 JP 288216/93; 17.11.1993 JP 288217/93; 26.07.1994 JP 173881/94
(71) Applicant: SRL, INC., Tachikawa-shi, Tokyo 190 (JP); SRL, INC., Shinjuku-ku, Tokyo 163 (JP)
(72) Inventor: Sakairi, Koji, Taito-ku, Tokyo 110 (JP); Hino, Yoshihiro, Taito-ku, Tokyo 110 (JP); Oka, Masanori, Shinjuku-ku, Tokyo 160 (JP); Takanashi, Naoki, Shinjuku-ku, Tokyo 160 (JP); Morimoto, Taeko, Shinjuku-ku, Tokyo 160 (JP)
(74) Representative: Tiedtke, Harro, Dipl.-Ing.

(57) **Abstract**

A physiologically active substance fixed sheet (1) (hereinafter the "sheet") comprising a water-insoluble base material (2) and a physiologically active substance fixed layer (3) comprised of a water-soluble resin and a physiologically active substance fixed through the water-soluble resin, formed on the surface of the base material all over or in part. The sheet has advantages that, when it is immersed in an aqueous medium such as a buffer solution, an aqueous solution of the physiologically active substance can be obtained in a short time, and also any constituents of the base material do not mix into the aqueous solution. Thus, the sheet can be used for various examinations including clinical examination. Moreover, in this sheet, the physiologically active substance is fixed on the base material through the water-soluble resin, and hence any containers for holding physiologically active substances are unnecessary and the substances can be weighed with more ease when used.

## Description

### BACKGROUND OF THE INVENTION

### 1. Field of the Invention

This invention relates to a physiologically active substance fixed sheet in which physiologically active substances such as antibodies, antigens or enzymes have been fixed or set stationary, and more particularly to a physiologically active substance fixed sheet utilized in various examinations including clinical examinations. It also relates to a process for its production, a method for its storage and a method of supplying a physiologically active substance through such a physiologically active substance fixed sheet.

### 2. Description of the prior art

In recent years, with progress of techniques for the preparation of monoclonal antibodies, immunological measures have been conventionally used in the screening of various diseases such as AIDS (acquired immune deficiency syndrome) and hepatitis. There are various reagents used for such examination, among which reagents for all sorts of physiologically active substances such as antigens, antibodies and enzymes are indispensable.

As means for circulating these reagents in a stable state, it has been common to use a method in which reagents are freeze-dried and formed into powders, or a method in which reagents are dissolved or suspended in buffers or in solutions containing protective materials and the resulting solutions or suspensions are refrigerated or freezed for their circulation.

These methods, however, require containers made of glass or the like. Such containers are of no further use once used, and are discarded. Since the containers themselves are expensive, these methods have been involved in economical disadvantages. Use of such containers also has brought about a disadvantage that space must be left for storage during circulation or before use.

Reagents supplied in this way are weighed when used, and put into use for experiments, examinations and so forth. In the case of freeze-dried powdery reagents, however, much labor must be taken for weighing the reagents, and also such reagents tend to remain in containers, having a disadvantage that they can be wastefull as they do.

Under such technical circumstances, there is a disclosure as to a method in which substances or materials having physiological activities are held in the form of a film or sheet so that they can be made easier to handle and are available without use of the containers (see U.S. Patent No. 5,098,893 and Japanese Patent Application Laid-open No. 5-124954).

More specifically, U.S. Patent No. 5,098,893 discloses a method for storage of materials in a composition comprising i) a carrier substance which is water-soluble or water-swellable and is in a glassy or rubbery amorphous state (e.g., a copolymer comprised of sucrose and epichlorohydrin) and ii) materials to be stored such as enzymes incorporated into such a carrier substance; the carrier substance being formed into a film. Japanese Patent Application Laid-open No. 5-124954 also discloses a sheet-like solid pharmaceutcal composition comprising a pharmacologically acceptable sheet-like carrier and a solution or suspension containing a material capable of having a physiological activity in a very small quantity (e.g., a hormone prepartion); the latter being printed, coated, sprayed or injected into the former.

According to the methods disclosed in these publications, the substances or materials having physiological activities are held in the form of a film or sheet, and hence any containers for holding such materials are unnecessary and the materials can be weighed with more ease when used. Thus, these are regarded as effective methods that can eliminate the disadvantages stated above.

Now, when the methods disclosed in these publications are actually applied in order to obtain physiologically active substance fixed sheets utilized in various examinations including clinical examinations, they have had the following problems.

First, in the storage of materials as disclosed in U.S. Patent No. 5,098,893, the materials to be stored such as enzymes are so made as to be held by a water-soluble or water-swellable carrier substance formed into a film, and hence the carrier substance is required to have a layer thickness which is large to a certain degree taking account of mechanical handling properties. Meanwhile, as the layer thickness of the carrier substance is made larger, it takes a longer time when immersed in an aqueous medium so as to be again dissolved, and when used the handling properties come into question. Accordingly, in the method disclosed in U.S. Patent No. 5,098,893, the carrier substance is brought into a glassy or rubbery amorphous state so that its solubility can be improved. Hence, conditions for manufacture are required to be set in a high precision, and if set incorrectly, the resulting filmy carrier substance may have a poor solubility. This method has such a problem.

As for the sheet-like solid pharmaceutical composition disclosed in Japanese Patent Application Laid-open No. 5-124954, the composition has a pharmacologically acceptable sheet-like carrier and hence it is unnecessary to make its layer thickness so large when the solution or suspension containing a material having a physiological activity is printed or coated to form a film thereof on the sheet-like carrier. Thus, the solubility mentioned above can not come into question so much compared with the method disclosed in U.S. Patent No. 5,098,893. However, since this sheet-like solid pharmaceutical composition is mainly designed for its oral administration, the pharmacologically acceptable sheet-like carrier is made of a water-soluble material or water-dispersible material as exemplified by an inorganic carrier such as cellulose or a derivative thereof, starch or a derivative thereof, a saccharide, titanium oxide, calcium carbonate, magnesium silicate or silicate anhydride. Hence, in the instance where the sheet-like carrier is made of a water-soluble material, the sheet-like carrier dissolves when printed or coated thereon with the above aqueous solution or suspension, so that it becomes difficult to deal with its manufacture. To avoid such a difficulty, spraying is employed. There, however, is a problem that films formed by the spraying are so thin that the above materials can not be fixed in an enough quantity. On the other hand, in the instance where the sheet-like carrier is made of a water-dispersible material, it becomes possible for the sheet-like carrier to be printed or coated thereon with the above aqueous solution or suspension. However, when the sheet-like solid pharmaceutical composition thus obtained is immersed in an aqueous medium so as to be dissolved, the materials constituting the sheet-like carrier mix into the aqueous medium in a dispersed state, so that it becomes difficult to apply this sheet-like solid pharmaceutical composition to the examinations including clinical examinations. This method has such a problem.

### SUMMARY OF THE INVENTION

The present invention was made taking note of such problems. An object thereof is to provide a physiologically active substance fixed sheet that can be used in various examinations including clinical examinations.

Another object of the present invention is to provide a process for producing such a physiologically active substance.

Still another object of the present invention is to provide a method for storage of such a physiologically active substance fixed sheet.

A further object of the present invention is to provide a method of supplying a physiologically active substance through such a physiologically active substance fixed sheet.

The physiologically active substance fixed sheet according to the present invention comprises a water-insoluble base material and a physiologically active substance fixed layer comprised of a water-soluble resin and a physiologically active substance fixed through the water-soluble resin, formed on the surface of the base material all over or in part.

In a preferred embodiment of the physiologically active substance fixed sheet, the physiologically active substance fixed layer is a plurality of physiologically active substance fixed layers separately arranged on the same plane of the water-insoluble base material, and the physiologically active substance comprises different kinds of physiologically active substances, and/or the same kind of physiologically active substances with different concentrations, having been each independently fixed therein.

The process for producing such a physiologically active substance according to the present invention comprises dissolving or dispersing a physiologically active substance in a solution of a water-soluble resin, and providing the resulting solution or dispersion to the surface of a water-insoluble base material all over or in part, followed by drying.

In the method for storage of such a physiologically active substance fixed sheet according to the present invention, the physiologically active substance fixed sheet is stored in an atmosphere of an inert gas, in an atmosphere of a vacuum, in an atmosphere of desiccation, in an atmosphere of an inert gas and desiccation, in an atmosphere of a vacuum and desiccation or in a sealed package.

In the method of supplying a physiologically active substance through such a physiologically active substance fixed sheet, the physiologically active substance fixed sheet is brought into contact with an aqueous medium to dissolve the physiologically active substance fixed layer.

### BRIEF DESCRIPTION OF THE DRAWINGS

Fig. 1 is a cross section of a physiologically active substance fixed sheet according to Example 1 of the present invention.

Fig. 2 is a cross section of a physiologically active substance fixed sheet according to another embodiment of the present invention.

Fig. 3 is a cross section of a physiologically active substance fixed sheet according to still another embodiment of the present invention.

Fig. 4 is a plan view of the physiologically active substance fixed sheet according to another embodiment of the present invention.

Fig. 5 is a plan view of a physiologically active substance fixed sheet according to Example 2 of the present invention.

Fig. 6 is a cross section of a physiologically active substance fixed sheet according to Example 3 of the present invention.

Fig. 7 is a plan view of a physiologically active substance fixed sheet constituted for use in clinical examination.

Fig. 8 is a graph showing the relationship between antigen quantity and absorbance.

Figs. 9A to 9F illustrate a process for enzyme immunoassay making use of a physiologically active substance fixed sheet according to the present invention.

Figs. 10A to 10J illustrate a process for enzyme immunoassay making use of a physiologically active substance fixed sheet according to the present invention.

Figs. 11A to 11G illustrate a process for enzyme immunoassay making use of a physiologically active substance fixed sheet according to the present invention.

### DESCRIPTION OF THE PREFERRED EMBODIMENTS

The present invention will be described below in detail.

The physiologically active substance fixed sheet according to the present invention is characterized by having a water-insoluble base material and a physiologically active substance fixed layer comprised of a water-soluble resin and a physiologically active substance fixed through the water-soluble resin, formed on the surface of the base material all over or in part.

The physiologically active substance fixed sheet according to the present invention has a water-insoluble base material and hence it is unnecessary to make its layer thickness so large as in the case of the sheet-like solid pharmaceutical composition disclosed in Japanese Patent Application Laid-open No. 5-124954.

Thus, when this physiologically active substance fixed sheet is immersed in an aqueous medium such as a buffer solution, the physiologically active substance fixed layer can dissolve in a short time and it becomes possible to readily obtain an aqueous solution of the physiologically active substance.

The above base material is water-insoluble, and hence the base material does not dissolve when the physiologically active substance fixed layer is formed by providing onto the base material a solution mainly composed of water containing the water-soluble resin and the physiologically active substance, and also any constituents of the base material do not mix into aqueous solution when the physiologically active substance fixed sheet produced is immersed in an aqueous medium to obtain an aqueous solution of the physiologically active substance.

Moreover, in this physiologically active substance fixed sheet, the physiologically active substance is fixed on the base material through the water-soluble resin (in the form of the physiologically active substance fixed layer), and hence as in the case of the means disclosed in U.S. Patent No. 5,098,893 and Japanese Patent Application Laid-open No. 5-124954 any containers for holding physiologically active substances are unnecessary and the substances can be weighed with more ease when used.

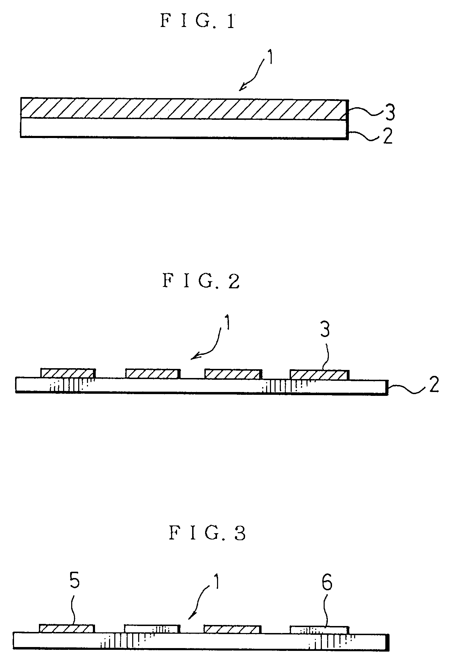

Fig. 1 illustrate an example of the constitution of the physiologically active substance fixed sheet according to the present invention. The physiologically active substance fixed sheet, denoted by reference numeral 1, is mainly comprised of a water-insoluble base material 2 and a physiologically active substance fixed layer 3 formed thereon by coating. As another embodiment thereof, the physiologically active substance fixed layer 3 may be formed in a pattern as shown in Fig. 2. In this case, the physiologically active substance fixed layer 3 formed in a pattern may be so formed as to comprise a plurality of physiologically active substance fixed layers in which the same kind of physiologically active substances with different concentrations have been each independently fixed. Alternatively, the physiologically active substance fixed layer may be so formed as to comprise a plurality of physiologically active substance fixed layers in which different kinds of physiologically active substances have been each independently fixed, e.g., as shown in Fig. 3, to comprise a physiologically active substance fixed layer 5 in which a physiologically active substance-(A) has been fixed and a physiologically active substance fixed layer 6 in which a physiologically active substance-(B) has been fixed, which are alternately formed by coating. The physiologically active substance fixed layer may also be so formed as to comprise a plurality of physiologically active substance fixed layers in which different kinds of physiologically active substances have been each independently fixed and a plurality of physiologically active substance fixed layers in which the same kind of physiologically active substances with different concentrations have been each independently fixed.

In the present invention, the physiologically active substance to be fixed can be exemplified by antibodies, antigens and enzymes. When the present physiologically active substance fixed sheet is applied to clinical examination such as examination on hepatitis, the substance may also include fluoroscein-labelled antibodies, fluorescein-labelled antigens and latex-labelled antibodies. Examples are by no means limited to these.

The water-insoluble base material on which the physiologically active substance is fixed may include materials such as polystyrene, polyethylene, polypropylene and polyethylene terephthalate, plastic films or plastic sheets formed of a composite of any of these materials, as well as glass, metals, plastic-coated products of these, and also plastic-coated papers. When plastic is used, untreated plastics are preferred. This is because the physiologically active substance having been fixed may tend to be adsorbed on the base material if any functional groups are produced on its surface upon corona treatment or plasma treatment applied thereon, and the amount of physiologically active substances participating in the reaction after they have been dissolved may be affected. Any of these materials may be used so long as they do not affect the activity and reaction of the physiologically active substance dissolved out when the physiologically active substance fixed is dissolved in an aqueous medium such as a buffer solution and put into use.

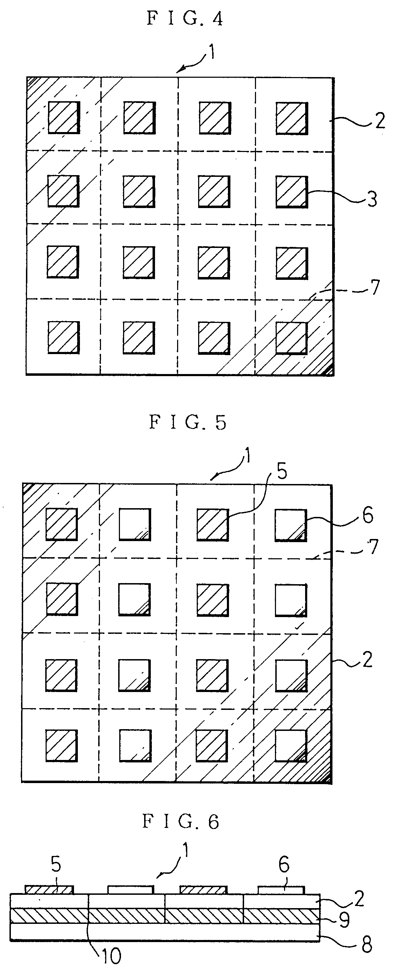

The base material 2 may also be pre-worked, e.g., provided with perforations 7 as shown in Figs. 4 and 5. As another example, as shown in Fig. 6, a sheet 8 having a pressure-sensitive adhesive 9 or the like may be stuck to the back of the base material 2 and only the base material 2 optionally together with the adhesive 9 may be half cut as shown by cut-off lines 10 so that the respective portions of the sheet are made readily separable. This can make the sheet easier to handle.

The water-soluble resin for fixing the physiologically active substance may include cellulose derivatives such as hydroxypropyl cellulose (HPC), carboxymethyl cellulose (CMC), methyl cellulose (MC), hydroxyethyl methyl cellulose (HEMC), hydroxyethyl cellulose (HEC) and hydroxypropyl methyl cellulose (HPMC), water-soluble natural polysaccharides such as sodium alginate, and also water-soluble synthetic resins such as polyvinyl alcohol (PVA), any of which may be used alone or in combination.

Use of polyvinyl alcohol (in particular, a completely saponified type) and carboxymethyl cellulose in combination has brought about good results preferable for maintaining the activity of physiologically active substances. The reason therefor is still unclear, and it is presumed that polyvinyl alcohol having a low oxygen permeability plays some role.

As a solvent for preparing a solution of the water-soluble resin, it is possible to use not only water but also an organic solvent such as ethanol or isopropanol in part as a mixed solvent. The organic solvent should preferably be used in an amount not more than 5 parts by weight based on 100 parts by weight of the total weight of the solvent. The solvent may be used so that the resin is in a content of from 0.1 to 20 parts by weight, and preferably from 1 part by weight to 5 parts by weight, based on 100 parts by weight of the solvent.

In the solution prepared by dissolving the water-soluble resin, the physiologically active substance is mixed, and dissolved or dispersed. The amount of the physiologically active substance to be mixed may vary depending on the viscosity and concentration of the physiologically active substance to be used, and can well be up to substantially the same amount as the amount of the resin in each water-soluble resin solution. Stated specifically, the physiologically active substance may be mixed so that the resin content comes to be from 1 part by weight to 100,000 parts by weight, and preferably from 50 parts by weight to 60,000 parts by weight, based on 1 part by weight of the physiologically active substance. Then the solution in which the water-soluble resin and the physiologically active substance have been mixed together with each other is provided to the surface of the base material by a suitable means such as coating or dropping. At this time, the solution may be coated over the entire surface of the base material, or may be so provided as to form a pattern on the surface. Also, solutions in which different kinds of physiologically active substances have been each independently mixed or solutions in which the same kind of physiologically active substances with different concentrations have been each independently mixed may be respectively provided to the surface of the same base material to form the pattern as previously shown in Fig. 2 or 3. Thereafter, the solution(s) provided to the surface is/are dried to form the physiologically active substance fixed layer(s) in which the physiologically active substance(s) has/have been fixed. Thus, the physiologically active substance fixed sheet according to the present invention can be produced.

The physiologically active substance fixed sheet as shown in Fig. 4 or 5 can be produced by patternwise providing to the surface of the base material previously partitioned by making cuts or perforations in dotted-lines, the water-soluble resin solutions in which the physiologically active substances have been dissolved or dispersed, followed by drying. The physiologically active substance fixed sheet as shown in Fig. 6 can be produced by patternwise providing to the surface of the base material to the back of which a sheet member has been laminated via a pressure-sensitive adhesive layer, the water-soluble resin solutions in which the physiologically active substances have been dissolved or dispersed, followed by drying, and thereafter cuts in lines may be made only in the base material.

The physiologically active substance fixed sheet thus produced can stably maintain the activities of physiologically active substances even when used as it is. In some instances, for example, the physiologically active substance in the physiologically active substance fixed layer is in a low concentration, the physiologically active substance to be fixed has originally a low activity, or the activities of physiologically active substances are required to be maintained over a long period of time. In such instances, the physiologically active substance fixed sheet may be packaged with a suitable packaging material for its storage, whereby it becomes possible to maintain the activities of physiologically active substances for a longer period.

The packaging material for such purpose may include, for example, packages of a triple-layer structure comprised of polyethylene terephthalate (PET)/aluminum (Al)/unstretched polypropylene (OPP), packages comprised of PET/Al/ polyethylene (PE), packages comprised of OPP/polyvinyl alcohol (PVA)/PE, and packages of a double-layer structure comprised of polyvinylidene chloride coated OPP (KOP)/PE.

When packaged, a disoxidant may be enclosed at the same time to remove oxygen inside the package, the inside of the package may be evacuated after the physiologically active substance fixed sheet has been enclosed, or the air inside the package may be replaced with carbon dioxide or an inert gas such as nitrogen, argon or helium to control oxygen concentration to be 2% or less, and preferably 0.5% or less. This makes it possible to maintain the activities of physiologically active substances for a much longer period. The same effect can be obtained also when the water vapor inside the package is removed by using a desiccating agent such as silica gel or by replacing it with dry gas or gases to provide a humidity of from about 0.1 to 0.003 mg/lit. Combination of any of these means can also achieve a more improvement in the storage stability.

When the physiologically active substance fixed sheet thus produced is used, the physiologically active substance fixed sheet itself is put into an aqueous medium such as a buffer solution and the physiologically active substance fixed therein is dissolved out to obtain an aqueous solution of physiologically active substance, which is then appropriately used for experiments or examinations.

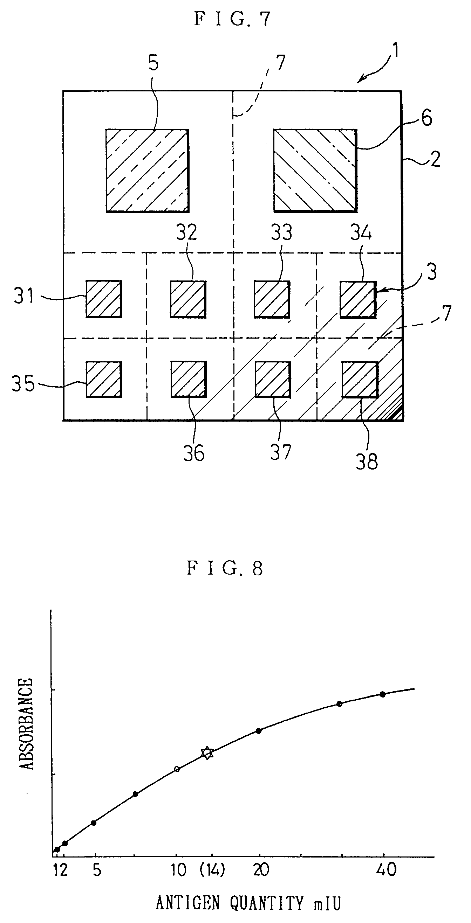

Fig. 7 is a plan view of an example of the physiologically active substance fixed sheet according to the present invention, constituted for use in clinical examination. This physiologically active substance fixed sheet, designated by reference numeral 1, is mainly comprised of a water-insoluble base material 2 provided with perforations 7, a physiologically active substance fixed layer 5 in which first antibodies have been fixed, a physiologically active substance fixed layer 6 in which second antibodies (labelled antibodies) have been fixed, and a physiologically active substance fixed layer 3 in which the same kind of antigens (e.g., AIDS virus antigens) with successively different concentrations (e.g., 1mIU, 5mIU, 10mIU, 15mIU, 20mIU, 25mIU, 30mIU, 40mIU) have been separately fixed. The physiologically active substance fixed layer 3 comprised of a plurality of physiologically active substance fixed layers 31 to 38 is designed for obtaining a calibration curve (a standard curve) (see Fig. 8) used when a clinical examination is made in accordance with the process described below.

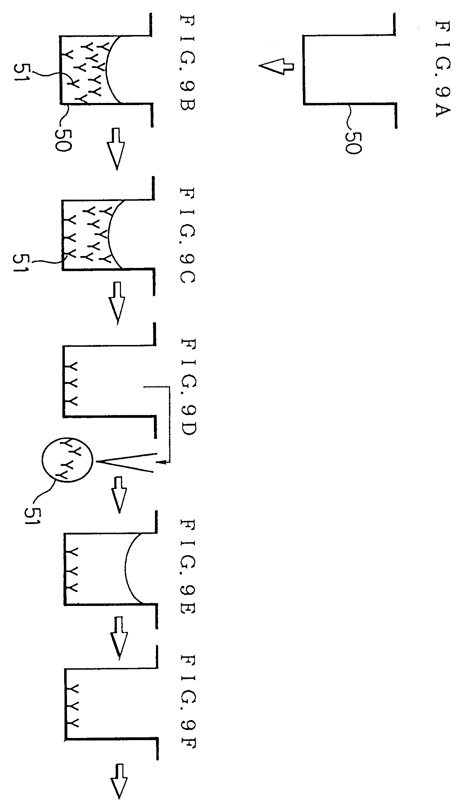

First, the base material 2 is cut off at the perforations 7 to separate the physiologically active substance fixed layer 5 in which first antibodies have been fixed, which is then put into a buffer solution to obtain an aqueous solution of the first antibodies. Next, the aqueous first-antibody solution thus obtained is supplied to a microplate well 50 (see Figs. 9A, 9B) so that a given quantity of the first antibodies 51 is physically adsorbed on the bottom (see Fig. 9C). Thereafter, the aqueous solution of the first antibodies 51 present in excess is discarded (see Fig. 9D) and also the microplate well 50 is washed (for example, about three times). Then the washings are discarded (see Figs. 9E, 9F).

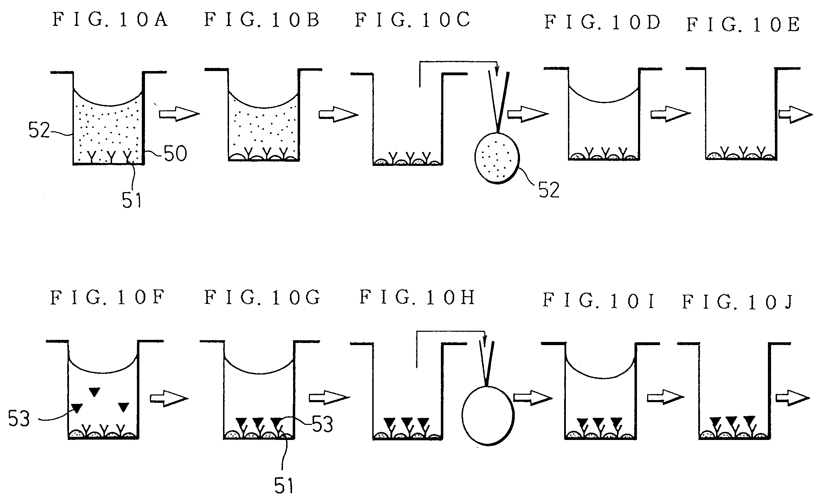

Next, a blocking solution 52 formed of a protein solution is fed into the microplate well 50 and adsorbed on the bottom portion of the microplate well 50 where no first antibodies 51 have been adsorbed, to block excess combining sites, and thereafter the blocking solution 52 is discarded (see Figs. 10A to 10C). Then, as shown in Figs. 10D to 10E, the well is washed about three times and also the washings are discarded.

Meanwhile, the physiologically active substance fixed layer 31 with a maximum concentration (e.g., 40mIU) is separated from the physiologically active substance fixed sheet 1 and this layer is put into a buffer solution to obtain an aqueous solution of antigens. Thereafter, the aqueous solution of antigens is fed into a microplate well 50 in which a given quantity of the first antibodies 51 have been adsorbed (see Fig. 10F). This introduction of antigens 53 causes an antigen-antibody reaction between the antibodies 51 and the antigens 53 in the microplate well 50, so that the antibodies 51 and the antigens 53 combine as shown in Fig. 10G. After this antigen-antibody reaction has been completed, the aqueous solution is discarded from the microplate well 50, the well is also washed about three times, and then the washings are discarded (see Figs. 10H to 10J).

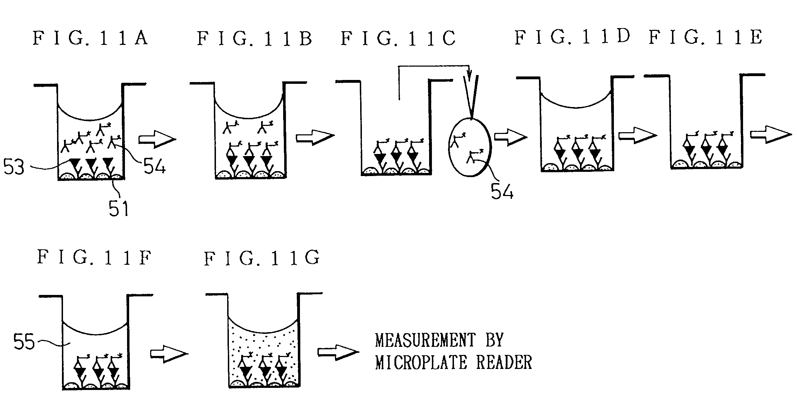

Next, from the physiologically active substance fixed sheet 1 the physiologically active substance fixed layer 6 in which second antibodies (labelled antibodies) have been fixed is separate, which is then put into a buffer solution to obtain an aqueous solution of enzyme labelled antibodies 54. Thereafter, this solution is fed into the above microplate well 50 (see Fig. 11A). This introduction of enzyme labelled antibodies 54 causes an antigen-antibody reaction between the antigens 53 and the enzyme labelled antibodies 54 in the microplate well 50 (see Fig. 11B). Next, excess enzyme labelled antibodies 54 are discarded, the well is also washed about three times, and then the washings are discarded (see Figs. 11C to 11E).

Next, a substrate solution 55 is fed into the microplate well 50 where the first antibodies 51, the antigens 53 and the enzyme labelled antibodies 54 have combined, to cause the enzyme labelled antibodies 54 to color (see Fig. 11F, 11G). The resulting sample is measured by means of a microplate reader to determine its absorbance, and the value obtained is plotted. Subsequently, the same procedure is repeated to successively plot absorbances at corresponding concentrations, using the physiologically active substance fixed layer 3 having a known concentration. Thus, the calibration curve as shown in Fig. 8 is obtained.

Then, a sample actually collected is analyzed according to the same procedure to obtain its absorbance, and the concentration of antigens in the sample can be measured from this absorbance through means of the calibration curve.

As described above, the physiologically active substance fixed sheet according to the present invention has the water-insoluble base material, and hence it is unnecessary to make the thickness of the physiologically active substance fixed layer so large. This brings about an advantage that, when this physiologically active substance fixed sheet is immersed in an aqueous medium such as a buffer solution, the physiologically active substance fixed layer can dissolve in a short time and it becomes possible to readily obtain an aqueous solution of the physiologically active substance.

In addition, the above base material is water-insoluble, and hence the base material does not dissolve when the physiologically active substance fixed layer is formed by providing onto the base material a solution mainly composed of water containing the water-soluble resin and the physiologically active substance, and also any constituents of the base material do not mix into aqueous solution when the physiologically active substance fixed sheet produced is immersed in an aqueous medium to obtain an aqueous solution of the physiologically active substance. Thus, there is an advantage that such a physiologically active substance fixed sheet can be used for various examinations including clinical examination.

Moreover, in this physiologically active substance fixed sheet, the physiologically active substance is fixed on the base material through the water-soluble resin, and hence any containers for holding physiologically active substances are unnecessary and the substances can be weighed with more ease when used.

The base material may also be partitioned by previously making cuts in dotted-lines, or by laminating to the back of the base material a sheet member via a pressure-sensitive adhesive layer and thereafter making cuts in lines only in the base material. This brings about an advantage that any necessary physiologically active substance fixed layers can be readily separated from the base material thus partitioned.

The physiologically active substance fixed sheet according to the present invention may also be stored in an atmosphere of an inert gas, in an atmosphere of a vacuum, in an atmosphere of desiccation, or the like, and there is an advantage that the the activities of physiologically active substances can be maintained for a longer period.

The present invention will be described below in greater detail by giving Examples.

### Example 1

A solution prepared by mixing 200 parts by weight of a polyclonal antibody solution (anti-human transferrin; immune animal: sheep; available from Wako Pure Chemical Industries, Ltd.) and 800 parts by weight of a water-soluble resin solution (methyl cellulose available from Shin-Etsu Chemical Co., Ltd.; viscosity: 16.5 cps; an aqueous 2.5% solution) was coated on a PET film (thickness: 75 µm; available from Diafoil Co.) by means of a coater so as to have a resin coating weight of 2 mg per 1 cm² after dried, further followed by drying at 20°C to obtain a physiologically active substance fixed sheet as shown in Fig. 1.

With regard to the physiologically active substance fixed sheet thus prepared, any changes in reactivity with antigens before and after drying, using human transferrin as antigens, were measured by quantitative precipitation in solution.

First, the physiologically active substance fixed sheet according to the present Example was cut off in 1 cm square and dissolved in a phosphate buffer physiological saline to obtain a sample for measurement. At this time, about 2 minutes were taken for the dissolution.

Meanwhile, antibodies having been not formed into a sheet (an anti-human transferrin solution that had been stored at 4°C) were used as a control. The respective samples were subjected to quantitative precipitin reaction in solution.

Then, following a known procedure, the precipitates were formed and collected, and colorimetrically analyzed by the Lowry process to compare their absorbance. As a result, the physiologically active substance fixed sheet according to the present Example showed no difference in absorbance before and after the drying and no change was seen in the reactivity with antigens.

The samples were also stored for 1 month in a 37°C thermostatic box, and their reactivities with antigens were examined in the same manner. As a result, there was no difference in absorbance between the physiologically active substance fixed sheet according to the present Example and the antibodies having been not formed into a sheet (the control) and it was ascertained that its reactivity was maintained in the sheet.

### Example 2

A physiologically active substance-(A) solution was prepared by mixing 200 parts by weight of a commercially available polyclonal antibody solution (anti-human transferrin; immune animal: sheep; available from Wako Pure Chemical Industries, Ltd.) and 800 parts by weight of a water-soluble resin solution (hydroxypropyl methyl cellulose available from Shin-Etsu Chemical Co., Ltd.; viscosity: 46.1 cps; an aqueous 2.5% solution).

A physiologically active substance-(B) solution was also prepared by mixing 200 parts by weight of the same polyclonal antibody solution and 9,800 parts by weight of a water-soluble resin solution (hydroxypropyl methyl cellulose available from Shin-Etsu Chemical Co., Ltd.; an aqueous 2.04% solution).

In the respective partitions of the base material 2 formed of a PET film, previously partitioned by making perforations 7 as shown in Fig. 5, the physiologically active substance-(A) solution and the physiologically active substance-(B) solution were alternately coated so as to have a resin coating weight of 2 mg per 1 cm² after dried, followed by drying to obtain a physiologically active substance fixed sheet as shown in Fig. 5, having physiologically active substance fixed layers 5 in which the physiologically active substance (A) had been fixed (i.e., physiologically active substance-(A) fixed layers) and physiologically active substance fixed layers 6 in which the physiologically active substance (B) had been fixed (i.e., physiologically active substance-(B) fixed layers).

In this physiologically active substance fixed sheet, antibodies with a concentration of 1/20 of that in the physiologically active substance-(A) fixed layer 5 were contained in the physiologically active substance-(B) fixed layer 6.

Next, the physiologically active substance-(A) fixed layers 5 and the physiologically active substance-(B) fixed layers 6 were separated from the physiologically active substance fixed sheet and were each dissolved in 1 ml of a phosphate buffer physiological saline to obtain samples for measurement, which were then subjected to quantitative precipitin reaction in solution. The time taken for the dissolution was about 2 minutes.

It was possible to use the physiologically active substance fixed sheet according to the present Example in the detection of antigens as effectively as antibody solutions having been not formed into sheets as in Example 1.

It also became possible to make examinations at different antibody concentrations since antibody solutions with different concentrations were simply prepared without any special operation of dilution.

### Example 3

As shown in Fig. 6, a sheet (woodfree paper) 8 having a pressure-sensitive adhesive 9 was stuck to the back of the base material 2 formed of a PET film.

Then, the same physiologically active substance-(A) solution and physiologically active substance-(B) solution as those prepared in Example 2 were alternately coated in the same manner as in Example 2, followed by drying. Thereafter, only the base material 2 was half cut as shown by cut-off lines 10 to partition the base material 2.

The physiologically active substance fixed sheet thus prepared was subjected to quantitative precipitin reaction in solution in the same manner as in Example 2. At this time, the separation of the physiologically active substance-(A) fixed layer 5 and the physiologically active substance-(B) fixed layer 6 was operable very simply and also it became possible to make adjustment with ease.

As a result, it was possible to use the sheet in the detection of antigens in the same manner as in Example 2.

### Example 4

Bags were made using a packaging material of a double-layer structure comprised of 20 µm thick polyvinylidene chloride coated unstretched polypropylene (KOP) and 40 µm thick polyethylene to obtain packages. Next, the physiologically active substance fixed sheet according to Example 1 was cut off in 1 cm square, and the resulting sheet pieces were enclosed in the packages. At this time, a disoxidant (trade name: AGELESS; available from Mitsubishi Gas Chemical Company, Inc.; for 100 ml head space) and 2 g of silica gel (available from Wako Pure Chemical Co., Ltd.) were enclosed together with the sheet, so that the oxygen concentration in each package was controlled to be 0.5%, and the water vapor, 0.1 mg/lit.

These were stored in a 37°C thermostatic box, and the reactivity with antigens of the sheet was examined with time by the method previously described. Meanwhile, that of another physiologically active substance fixed sheet according to Example 1 but stored in a 37°C thermostatic box without any particular packaging was also examined as a control.

As a result, the physiologically active substance fixed sheet according to Example 1 but stored in a 37°C thermostatic box without packaging maintained the reactivity until the lapse of one month, but thereafter gradually caused a lowering of activity and became almost inactive in three months. On the other hand, the packaged physiologically active substance fixed sheet according to the present Example caused no such lowering of activity and was found to have maintained its activity over a period of seven months.

### Example 5

A solution prepared by mixing 200 parts by weight of an enzyme solution (prepared by dissolving 1 part by weight of horseradish peroxidase available from Toyobo Co. Ltd. in 1,000 parts by weight of 0.1 mol phosphate buffer solution of pH 7.0) and 800 parts by weight of a water-soluble resin solution [a solution containing 3% by weight of methyl cellulose (available from Shin-Etsu Chemical Co., Ltd.; viscosity: 16.5 cps) and 2% by weight of hydroxypropyl cellulose (available from Shin-Etsu Chemical Co., Ltd.; viscosity: 8 cps)] was coated on a PET film (thickness: 75 µm; available from Diafoil Co.) by means of a coater so as to have a resin coating weight of 4 mg per 1 cm² after dried, further followed by drying at 20°C to obtain a physiologically active substance fixed sheet as shown in Fig. 1.

Next, bags were made using a packaging material of a triple-layer structure comprised of 12 µm thick polyethylene terephthalate (PET), 9 µm thick aluminum and 50 µm thick unstretched polypropylene to obtain packages. Next, the above physiologically active substance fixed sheet was cut off in 1 cm square, and the resulting sheet pieces were enclosed in the packages. At this time, a disoxidant (trade name: AGELESS; available from Mitsubishi Gas Chemical Company, Inc.; for 100 ml head space) and 2 g of silica gel (available from Wako Pure Chemical Co., Ltd.) were enclosed together with the sheet, so that the oxygen concentration in each package was controlled to be 0.5%, and the water vapor, 0.1 mg/lit.

These were stored in a 25°C thermostatic box, and the enzymatic activity of the physiologically active substance fixed sheet during storage was observed. Meanwhile, another physiologically active substance fixed sheet according to the present Example but stored at 25°C without packaging was also prepared as a control. Then the physiologically active substance fixed sheet that had been enclosed in the package and the physiologically active substance fixed sheet not packaged were each dissolved using a 50 ml of 0.1 mol phosphate buffer solution (pH 7.0). The time taken for the dissolution was about 5 minutes. Using these as samples for measurement, the enzymatic activities were measured by a known method making use of tetramethyl benzidine and hydrogen peroxide as coloring substrates.

As a result, the physiologically active substance fixed sheet according to the present Example but stored at 25°C without packaging caused a decrease with time in coloring and showed no coloring on 28th day, resulting in inactivation of enzymes. On the other hand, the packaged physiologically active substance fixed sheet according to the present Example caused no changes immediately after its preparation and thereafter, and was found to have maintained the enzymatic activity over a period of seven months.

### Example 6

A solution prepared by mixing 200 parts by weight of a human transferrin solution (prepared by dissolving 5 parts by weight of human transferrin available from Wako Pure Chemical Industries, Ltd. in 1,000 parts by weight of 0.1 mol phosphate buffer solution of pH 7.0) and 800 parts by weight of a water-soluble resin solution [a solution containing 4% by weight of hydroxypropyl cellulose (available from Nippon Soda Co., Ltd.; viscosity: 8.5 cps) and 6% by weight of carboxymethyl cellulose (available from Dai-ichi Kogyo Seiyaku Co., Ltd.; viscosity: 150 cps)] was dropped on a PET film (thickness: 75 µm; available from Diafoil Co.) by means of a dispenser so as to have a resin weight of 8 mg per spot after dried, further followed by drying at 20°C to obtain a physiologically active substance fixed sheet as shown in Fig. 2. After dried, each spot contained 100 µg of human transferrin.

With regard to the physiologically active substance fixed sheet thus prepared, any changes in reactivity with human transferrin antibodies before and after drying, using anti-human transferrin (immune animal: sheep; available from Wako Pure Chemical Industries, Ltd.) as antibodies, were measured by quantitative precipitation in solution.

More specifically, one sheet was cut off and dissolved in 2 ml of a 0.1 mol phosphate buffer solution (pH 7.0) to obtain a sample for measurement. The time taken for the dissolution was about 3 minutes.

Meanwhile, a human transferrin powder that had been stored at 4°C was used as a control. A solution thereof with the same concentration was prepared, and, together with the above sample, subjected to quantitative precipitin reaction in solution.

Then, following a known procedure, the precipitates were formed and collected, and colorimetrically analyzed by the Lowry process to compare their absorbance. As a result, the physiologically active substance fixed sheet according to the present Example showed no difference in absorbance before and after the drying and no change was seen in the reactivity with antibodies.

The samples were also stored for 1 month in a 25°C thermostatic box, and their reactivities with antibodies were examined in the same manner. As a result, there was no difference in absorbance between the physiologically active substance fixed sheet according to the present Example and the antigens having been not formed into a sheet (the control) and it was ascertained that its reactivity, i.e., the reactivity with antibodies was maintained in the sheet.

### Example 7

Human chorionic gonadotropin (hereinafter "HCG") was selected as antigens, and antibodies were prepared by a known method using mice as immune animals. Next, the antibodies thus obtained were allowed to react with horseradish peroxidase (available from Toyobo Co. Ltd.) by a known method to prepare enzyme labelled antibodies (i.e., a combination of the above antibodies with the horseradish peroxidase). Then the enzyme labelled antibodies were dissolved in a 0.1 mol phosphate buffer solution (pH 0.7) and adjusted to have a protein concentration of 60 µg/ml. Thus, an enzyme labelled antibody solution was thus obtained.

Next, a solution prepared by mixing 200 parts by weight of this enzyme labelled antibody solution and 800 parts by weight of a water-soluble resin solution [an aqueous solution containing 0.3% by weight of partially saponified polyvinyl alcohol (available from Kanto Chemical Co., Inc.; degree of saponification: 88%; degree of polymerization: about 500) and 2.2% by weight of carboxymethyl cellulose (available from Dai-ichi Kogyo Seiyaku Co., Ltd.; viscosity: 150 cps)] was dropped on a PET film (thickness: 75 µm; available from Diafoil Co.) by means of a dispenser so as to have a resin weight of 2 mg per spot after dried, further followed by drying at 20°C to obtain a physiologically active substance fixed sheet as shown in Fig. 2.

Next, bags were made using a packaging material of a triple-layer structure comprised of 12 µm thick polyethylene terephthalate (PET), 9 µm thick aluminum and 50 µm thick unstretched polypropylene to obtain packages. Next, the above physiologically active substance fixed sheet was cut off for each spot, and the resulting sheet pieces were enclosed in the packages. At this time, a disoxidant (trade name: AGELESS; available from Mitsubishi Gas Chemical Company, Inc.; for 100 ml head space) and 2 g of silica gel (available from Wako Pure Chemical Co., Ltd.) were enclosed together with the sheet, so that the oxygen concentration in each package was controlled to be 0.5%, and the water vapor, 0.1 mg/lit.

These were stored in a 12°C thermostatic box, and immune response during storage was observed. Meanwhile, a an anti-HCG enzyme labelled antibody solution stored at 4°C was also prepared as a control, and its activity was measured by enzyme immunoassay making use of HCG as antigens.

More specifically, in each well of a microplate made of polystyrene, the antibodies were brought into a solid phase using an anti-HCG solution (available from Hycor Biomedical Inc.). Then, after blocking carried out by a known method, a solution of HCG (available from Chemicon International Inc.) diluted with a phosphate buffer solution was put into each well of the microplate in a given quantity to cause antigen-antibody reaction. After the reaction, excess HCG was removed, and the wells were washed.

Then the physiologically active substance fixed sheet was dissolved in a 50 ml of 0.1 mol phosphate buffer solution (pH 7.0). Here, the time taken for the dissolution was about 3 minutes. The resulting solution was put into each well of the microplate in a given quantity to cause it to react with HCG having combined with the solid phase antibodies in the wells.

After they were allowed to react with each other for 2 hours, unreacted enzyme labelled antibodies were removed and the wells were washed. The reacted enzyme labelled antibodies were further caused to color by a known method making use of tetramethyl benzidine and hydrogen peroxide as coloring substrates, and the coloring was stopped using 2N sulfuric acid. Then the absorbance was measured at 450 nm to compare the degree of coloring.

As a result, the physiologically active substance fixed sheet according to the present Example showed no difference in coloring before and after the drying, and maintained the reactivity with antigens and the enzymatic activity also after the drying.

Even after storage for 3 months, the same results were also obtained at the same concentration as the control enzyme labelled antibodies stored at 4°C, and the activities of the enzyme labelled antibodies were maintained.

A physiologically active substance fixed sheet (hereinafter the "sheet") comprising a water-insoluble base material and a physiologically active substance fixed layer comprised of a water-soluble resin and a physiologically active substance fixed through the water-soluble resin, formed on the surface of the base material all over or in part. The sheet has advantages that, when it is immersed in an aqueous medium such as a buffer solution, an aqueous solution of the physiologically active substance can be obtained in a short time, and also any constituents of the base material do not mix into the aqueous solution. Thus, the sheet can be used for various examinations including clinical examination. Moreover, in this sheet, the physiologically active substance is fixed on the base material through the water-soluble resin, and hence any containers for holding physiologically active substances are unnecessary and the substances can be weighed with more ease when used.

## Claims

1. A physiologically active substance fixed sheet comprising a water-insoluble base material and a physiologically active substance fixed layer comprised of a water-soluble resin and a physiologically active substance fixed through the water-soluble resin, formed on the surface of the base material all over or in part.

2. The physiologically active substance fixed sheet according to claim 1, wherein said physiologically active substance fixed layer comprises a plurality of physiologically active substance fixed layers separately arranged on the same plane of the water-insoluble base material, and said physiologically active substance comprises different kinds of physiologically active substances having been each independently fixed therein.

3. The physiologically active substance fixed sheet according to claim 1, wherein said physiologically active substance fixed layer comprises a plurality of physiologically active substance fixed layers separately arranged on the same plane of the water-insoluble base material, and said physiologically active substance comprises the same kind of physiologically active substances with different concentrations, having been each independently fixed therein.

4. The physiologically active substance fixed sheet according to claim 1, wherein said physiologically active substance fixed layer comprises a plurality of physiologically active substance fixed layers separately arranged on the same plane of the water-insoluble base material, and said physiologically active substance comprises different kinds of physiologically active substances and the same kind of physiologically active substances with different concentrations, having been each independently fixed therein.

5. The physiologically active substance fixed sheet according to claim 4, wherein said different kinds of physiologically active substances are comprised of an antibody or antigen and a labelled antibody or labelled antigen thereof, and the same kind of physiologically active substances with different concentrations are comprised of an antigen or antibody.

6. The physiologically active substance fixed sheet according to any one of claims 1 to 5, wherein said water-soluble resin is comprised of a mixture of polyvinyl alcohol and carboxymethyl cellulose.

7. The physiologically active substance fixed sheet according to any one of claims 1 to 5, wherein said base material is partitioned by making cuts in dotted-lines; said physiologically active substance fixed layer being provided on each base material thus partitioned.

8. The physiologically active substance fixed sheet according to any one of claims 1 to 5, wherein said base material is provided on the back thereof with a sheet member laminated via a pressure-sensitive adhesive layer and is partitioned by making cuts in lines only in the base material; said physiologically active substance fixed layer being provided on each base material thus partitioned.

9. A process for producing a physiologically active substance fixed sheet comprising a water-insoluble base material and a physiologically active substance fixed layer comprised of a water-soluble resin and a physiologically active substance fixed through the water-soluble resin, formed on the surface of the base material all over or in part;
said process comprising dissolving or dispersing a physiologically active substance in a solution of a water-soluble resin, and providing the resulting solution or dispersion to the surface of a water-insoluble base material all over or in part, followed by drying.

10. The process for producing the physiologically active substance fixed sheet according to claim 9, wherein said physiologically active substance comprises different kinds of physiologically active substances each independently dissolved or dispersed in said solution of a water-soluble resin; the resulting solutions or dispersions being independently provided to the surface of said base material.

11. The process for producing the physiologically active substance fixed sheet according to claim 9, wherein said physiologically active substance comprises the same kinds of physiologically active substances with different concentrations each independently dissolved or dispersed in said solution of a water-soluble resin; the resulting solutions or dispersions being independently provided to the surface of said base material.

12. The process for producing the physiologically active substance fixed sheet according to claim 9, wherein said base material is partitioned by making cuts in dotted-lines; said solution or dispersion being provided to the surface of each base material thus partitioned.

13. The process for producing the physiologically active substance fixed sheet according to claim 9, wherein said base material is provided on the back thereof with a sheet member laminated via a pressure-sensitive adhesive layer; said solution or dispersion being provided to the surface of the base material, which is then partitioned by making cuts in lines only in the base material after drying.

14. A method for storage of a physiologically active substance fixed sheet comprising a water-insoluble base material and a physiologically active substance fixed layer comprised of a water-soluble resin and a physiologically active substance fixed through the water-soluble resin, formed on the surface of the base material all over or in part;
wherein said physiologically active substance fixed sheet is stored in an atmosphere from which oxygen or water content has been removed.

15. The method for storage of a physiologically active substance fixed sheet according to claim 14, wherein said atmosphere is an atmosphere of an inert gas.

16. The method for storage of a physiologically active substance fixed sheet according to claim 14, wherein said atmosphere is an atmosphere of a vacuum.

17. The method for storage of a physiologically active substance fixed sheet according to claim 14, wherein said atmosphere is an atmosphere of desiccation.

18. The method for storage of a physiologically active substance fixed sheet according to claim 14, wherein said atmosphere is an atmosphere of an inert gas and desiccation.

19. The method for storage of a physiologically active substance fixed sheet according to claim 14, wherein said atmosphere is an atmosphere of a vacuum and desiccation.

20. The method for storage of a physiologically active substance fixed sheet according to claim 14, wherein said physiologically active substance fixed sheet is stored in a sealed package.

21. A method of supplying a physiologically active substance through a physiologically active substance fixed sheet comprising a water-insoluble base material and a physiologically active substance fixed layer comprised of a water-soluble resin and a physiologically active substance fixed through the water-soluble resin, formed on the surface of the base material all over or in part;
wherein said physiologically active substance fixed sheet is brought into contact with an aqueous medium to dissolve the physiologically active substance fixed layer.
